(19) Europäisches Patentamt
European Patent Office
Office européen des brevets

(11) Numéro de publication: **0 044 228**
**B1**

(12) **FASCICULE DE BREVET EUROPEEN**

(45) Date de publication du fascicule du brevet: **13.02.85**

(51) Int. Cl.⁴: **C 08 B 37/10**, A 61 K 31/725

(21) Numéro de dépôt: **81400727.4**

(22) Date de dépôt: **08.05.81**

(54) **Esters d'héparine utilisables pour la préparation de médicaments, et procédés pour leur préparation.**

(30) Priorité: **14.05.80 FR 8010792**

(43) Date de publication de la demande:
**20.01.82 Bulletin 82/03**

(45) Mention de la délivrance du brevet:
**13.02.85 Bulletin 85/07**

(84) Etats contractants désignés:
**BE CH DE FR GB IT LI LU NL SE**

(56) Documents cités:
**DE-A-1 768 806**
**FR-A-2 100 735**
**FR-A-2 267 111**
**FR-M- 2 739**

**CHEMICAL ABSTRACTS, vol. 77, no. 19, 6 novembre 1972, page 7 réf. 121990w Columbus, Ohio, US I. DANISHEFSKY et al.: "Heparin derivatives prepared by modification of the uronic acid carboxyl groups"**

(73) Titulaire: **PHARMUKA LABORATOIRES**
**35 Quai du Moulin de Cage**
**F-92231 Gennevilliers (FR)**

(72) Inventeur: **Mardiguian, Jean**
**3, Villa Christine**
**F-94210 La Varenne Saint-Hilaire (FR)**

(74) Mandataire: **Gaumont, Robert et al**
**RHONE-POULENC RECHERCHES Service**
**Brevets Pharma 25, Quai Paul Doumer**
**F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 044 228 B1

**Description**

La présente invention a pour objet de nouveaux esters d'héparine et leurs sels alcalins, alcalino-terreux, de magnésium, d'ammonium quaternaire ou d'amine. Ces composés sont utilisables en tant que produits intermédiaires pour la préparation de médicaments.

Il est déjà connu (cf. brevet anglais 973 894 et brevet français de médicament 2739.M) des esters méthylique et benzylique totaux de l'héparins. Il est également connu (cf brevet anglais 1 501 095 at brevet français 2 150 724) des esters d'héparine résultant du remplacement partiel ou total des groupes carboxyliques de la structure de l'héparine par des groupes

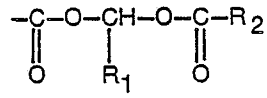 ($R_1$ = H ou $CH_3$; $R_2$ = alkyl 1–4 C),

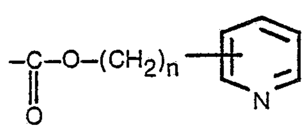 (n = 1, 2 ou 3), 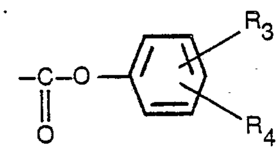 ($R_3$, $R_4$ = H, $NO_2$, COOH, alkyloxycarbonyl), 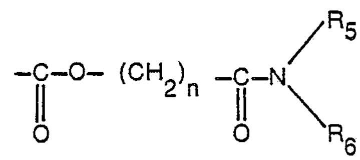 (n = 1, 2 ou 3; $R_5$, $R_6$ = alkyl 1–4.C).

Ces esters sont pour la plupart des agents anticoagulants à action prolongée.

Les esters d'héparine selon la présente invention dérivent de l'héparine par le remplacement partiel des groupes carboxyliques de la structure de l'héparine par des groupes de formule:

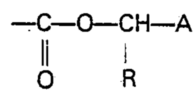 (I)

dans laquelle R est un atome d'hydrogène ou un groupe méthyle et A est un atome d'hydrogène ou un groupe méthyle, phényle,

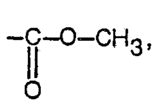, 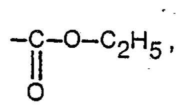, CN ou 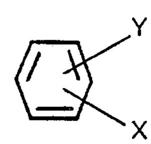,

X représentant un atome de chlore ou un groupe nitro, alkyle 1 à 4 C ou méthoxy et Y représentant un atome d'hydrogène ou de chlore, le remplacement partiel correspondant à un taux d'estérification de 10% à 90% des groupes carboxyliques dans le cas où A est un atome d'hydrogène ou un groupe méthyle ou phényle, et sont des esters sélectifs dans lesquels sont estérifiés, partiellement ou totalement, soit uniquement les groupes carboxyliques des chaînons acide D-glucuronique, soit uniquement les groupes carboxyliques des chaînons acide D-glucuronique et acide L-iduronique non sulfaté, soit encore uniquement les groupes carboxyliques des chaînons acide L-iduronique non sulfaté et acide L-iduronique sulfaté, soit enfin uniquement les groupes carboxyliques des chaînons acide L-iduronique sulfaté.

Dans la formule (I) ci-dessus, R est de préférence un atome d'hydrogène et A un groupe

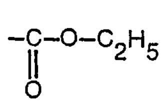, CN, 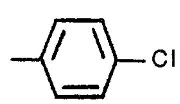 (p-chlorophényl) ou 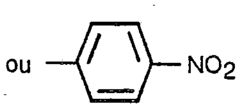 (p-nitrophényl).

Les sels alcalins, alcalino-terreux, de magnésium, d'ammonium quaternaire et d'amine des esters précédents font également partie de l'invention. Comme sels alcalins on peut citer en particulier les sels de sodium et comme sels alcalino-terreux les sels de calcium. Comme sels d'amine on peut citer les sels d'amine tertiaire, en particulier les sels de triéthylamine, pyridine ou imidazole. Comme sels d'ammonium quaternaire on peut citer les sels d'alkyl- ou d'aralkylammonium quaternaire à longue chaîne, en particulier les sels de dodécyltriméthylammonium et les sels de benzéthonium, et les sels de cétylpyridinium.

Les esters d'héparine sélectifs selon l'invention dans lesquels sont estérifiés, partiellement ou totalement, soit uniquement les groupes carboxyliques des chaînons acide D-glucuronique, soit uniquement les groupes carboxyliques des chaînons acide D-glucuronique et acide L-iduronique non sulfaté, sont obtenus par réaction d'un dérivé halogéné de formule:

Hal—CH—A (III)
|
R

dans laquelle Hal désigne un atome de chlore, de brome ou d'iode et R et A ont les mêmes significations que dans la formule (I), avec un sel acide d'ammonium quaternaire de l'héparine dans lequel sont salifiés, outre les groupes sulfate, soit uniquement les groupes carboxyliques des chaînes acide D-glucuronique, soit uniquement les groupe carboxyliques des chainons acide D-glucuronique et acide L-iduronique non sulfaté, les autres groupes carboxyliques étant sous forme acide. Cette rętion est effectue5e, en solution ou en suspension dans un solvant inerte, à une température comprise entre −20°C et +60°C. Comme solvants inertes, on peut citer en particulier le diméthylformamide, le chloroforme, l'hexaméthylphosphorylamide, le chlorure de méthylène, le diméthylsulfoxyde, le tétrahydrofuranne et l'acétone. Le taux d'estérification des groupes carboxyliques dépend des conditions opératoires (nature du dérivé halogéné de formule (III) et du solvant, durée de réaction, proportion des réactifs, température, etc...)

Les esters d'héparine obtenus par le procédé ci-dessus, dont les groupes sulfate sont sous forme sulfate d'ammonium quaternaire, peuvent être transformés en sels alcalins, alcalino-terreux, de magnésium, ou d'amine ou en esters d'héparine non salifiés par des méthodes connues en soi. Par exemple, pour préparer les sels alcalins, alcalino-terreux ou de magnésium, on fait agir sur l'ester d'héparine sous forme sulfate d'ammonium quaternaire l'acétate correspondant. Pour préparer les sels d'amine, on fait agir sur l'ester d'héparine sous forme sulfate d'ammonium quaternaire l'acétate de sodium de façon à former le sel de sodium, puis on fait passer ce sel de sodium sur une résine échangeuse de cations sulfonique sous forme $H^+$ de façon à former l'acide, salifie ledit acide par l'amine et isole le produit formé par lyophilisation ou précipitation par addition d'acétone.

Les sels acides d'ammonium quaternaire de l'héparine, dans lequels sont salifiés, outre les groupes sulfate, uniquement les groupes carboxyliques des chaînons acide D-glucuronique, sont obtenus par action d'un sel d'ammonium quaternaire sur l'héparine, en milieux aqueux, à un pH compris entre 3 et 4.

Les sels acides d'ammonium quaternaire de l'héparine, dans lesquels sont salifiés, outre les groupes sulfate, uniquement les groupes carboxyliques des chaînons acides D-glucornique et L-iduronique non sulfaté, sont obtenus par action d'un sel d'ammonium quaternaire sur l'héparine, en milieu aqueux, à un pH suffisamment bas pour former le sel d'ammonium quaternaire de l'héparine dans lequel seuls sont salifiés les groupes sulfate (en pratique pH 2 à 2,5), puis neutralisation sélective des groupes carboxyliques des chaînons acide D-glucuronique et acide L-iduronique non sulfaté du produit ainsi obtenu par addition d'une quantité calculée d'hydroxyde d'ammonium quaternaire en milieu diméthylformamide. Cette quantité est calculée en se basant sur la courbe de neutralisation d'une prise de poids déterminé dudit produit en milieu diméthylformamide.

Les esters d'héparine sélectifs selon l'invention dans lesquels sont estérifiés, partiellement ou totalement, soit uniquement les groupes carboxyliques des chaînons acide L-iduronique sulfaté soit uniquement les groupes carboxyliques des chaînons acide L-iduronique non sulfaté et acide L-iduronique sulfaté sont préparés par action d'un alcool de formule:

HO—CH—A (II)
|
R

dans laquelle R et A ont les même significations que dans la formule (I), sur l'héparine, en milieu aqueux, en présence d'un agent de condensation du type carbodiimide soluble dans l'eau tel que par exemple l'éthyl-1 (diméthylamino-3 propyl)-3 carbodiimide, le pH du milieu étant ajusté à une valeur comprise dans l'intervalle 3,5—4,5 dans le premier cas et à une valeur comprise dans l'intervalle 2—3 dans le deuxième cas. Comme alcool de formule (II) utilisable on peut citer en particulier le méthanol et l'éthanol, auquel cas on obtient respectivement des esters d'héparine sélectifs méthylique et éthylique.

Les composés selon l'invention subissent, lorsqu'on les soumet à l'action d'une solution aqueuse d'hydroxyde de sodium 0,1N à 0,6N à la température de 20°C à 60°C ou à l'action d'une base organique telle que le diaza-1,5-bicyclo[4.3.0]nonène-5 au sein du chlorure de méthylène, une dépolymérisation qui conduit à des mélanges de polysaccharides sulfatés ayant un poids moléculaire moyen inférieur à celui de

l'héparine. Ces mélanges sont des agents anticoagulants et antithrombotiques utiles pour la prévention et le traitement des thromboses.

Les exemples suivants illustrent l'invention sans la limiter. Le sel de sodium d'héparine utilisé comme produit de départ dans ces exemples correspond à une héparine de muqueuse de porc ayant un poids moléculaire moyen en poids de 16 000 daltons, un pouvoir rotatoire spécifique en solution aqueuse à 20°C de +44° et une activité anticoagulante codix de 180 u.i/mg.

Exemple 1

A une solution de 10 g d'héparine (sel de sodium) dans 40 ml d'eau on ajoute 2,5 ml d'acide acétique puis, lentement et sous agitation, 150 ml d'une solution aqueuse à 10% de chlorure de benzéthonium. Le précipité formé est recueilli par centrifugation, lavé à l'eau et séché. On obtient 19,67 g d'héparinate acide de benzéthonium.

On dissout 5 g du produit précédent dans 100 ml de diméthylformamide et ajoute 5 g de chlorure de (chloro-4) benzyle. On laisse en contact 48 heures à température ambiante, puis on ajoute 100 ml d'une solution à 10% d'acétate de sodium dans le méthanol. Le précipité formé est isolé par filtration, lavé au méthanol et séché sous vide. On obtient ainsi 2,11 g d'ester (chloro-4) benzylique d'héparine, sous forme de sel de sodium.

Caractéristiques du produit obtenu:

. Taux d'estérification des groupes carboxyliques: 22%
. Spectre d'absorption dans l'ultra-violet d'un solution aqueuse à 50 µg/ml:
. Maxima à 196 nm et 220 nm
. Densité optique à 220 nm pour une épaisseur de 1 cm: 0,120

Exemple 2

A une solution de 10 g d'héparine (sel de sodium) dans 40 ml d'eau on ajoute 2,5 ml d'acide acétique puis, lentement et sous agitation, 150 ml d'une solution aqueuse à 10% de chlorure de benzéthonium. Le précipité formé est recueilli par centrifugation, lavé à l'eau et séché. On obtient 19,67 g d'héparinate acide de benzéthonium.

On dissout 5 g du produit précédent dans 25 ml de dichlorométhane et ajoute 2,2 ml d'iodure de méthyle. La solution est conservée à l'abride la lumière pendant 24 heures, à la température ambiante. On ajoute 75 ml d'une solution d'acétate de sodium à 10% dans le méthanol. Le précipité formé est insolé par filtration, lavé au méthanol et séché sous vide. Onobtient 2 g d'ester méthylique d'héparine, sous forme de sel de sodium (taux d'estérification des groupes carboxyliques: 22%).

Exemple 3

A une solution de 10 g d'héparine (sel de sodium) dans 40 ml d'eau on ajoute 2,5 ml d'acide formique puis, lentement et sous agitation, 150 ml d'une solution aqueuse à 10% de chlorure de benzéthonium. On recueille le précipité par centrifugation, le lave à l'eau et le sèche sous vide. On obtient ainsi 20,5 g d'héparinate acide de benzéthonium. On dissout 10 g du produit précédent dans 200 ml de diméthylformamide, puis on ajoute 27,4 ml d'une solution 0,1 N d'hydroxyde de tétrabutylammonium dans un mélange n-propanol/méthanol.

Après addition de 10 g de chlorure de (chloro-4) benzyle on abandonne cinq jours à température ambiante. On ajoute 250 ml d'une solution à 10% d'acétate de sodium dans le méthanol. On isole par filtration 4,50 g d'ester (chloro-4) benzylique d'héparine, sous forme de sel de sodium.

Caractéristiques du produit obtenu:

. Taux d'estérification des groupes carboxyliques: 45%
. Spectre d'absorption dans l'ultra-violet d'une solution aqueuse à 50 µg/ml:
. Maxima à 196 nm et 220 nm
. Densité optique à 220 nm pour une épaisseur de 1 cm: 0,265

Exemples 4 á 8

A une solution de 0,600 g d'héparine (sel de sodium) dans 7 ml d'eau, dont le pH a été ajusté à la valeur désirée (cf tableau ci-dessous) par addition d'une solution aqueuse N d'acide chlorhydrique, on ajoute 0,300 g d'éthyl-1 (diméthylamino-3 propyl)-3 carbodiimide.

Après une heure de contact à la température ambiante, on ajoute 2,5 ml d'une solution aqueuse de chlorure de sodium à 280 g/l, puis 15 ml de méthanol. On recueille le précipité formé per filtration, la lave au méthanol et le sèche sous vide. On obtient ainsi des esters méthylique d'héparine, sous forme de sel de sodium, dont le taux d'estérification des groupes carboxyliques est donné dans le tableau ci-dessous.

| Exemple | pH de la solution aqueuse d'héparine | Poids d'ester obtenu | Taux d'estérification |
|---|---|---|---|
| 4 | 2,5 | 0,565 g | 77 % |
| 5 | 3 | 0,647 g | 70 % |
| 6 | 3,5 | 0,527 g | 50 % |
| 7 | 4 | 0,545 g | 31 % |
| 8 | 4,5 | 0,550 g | 19 % |

Dans le cadre de la présente demande, le dalton est défini comme étant égal au seizième de la masse d'un atome d'oxygène, soit $1{,}65 \times 10^{-24}$ gramme (cf. A. MANUILA, L. MANUILA, M. NICOLE, H. LAMBERT, Dictionnaire Français de Médecine et de Biologie, Tome I, 1970, p. 744).

**Revendications**

1. Esters d'héparine dérivant de l'héparine par le remplacement partiel des groupements carboxyliques de l'héparine par des groupes de formule:

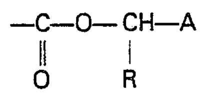
(I)

dans laquelle R est un atome d'hydrogène ou un groupe méthyle et A est un atome d'hydrogène ou un groupe méthyle, phényle,

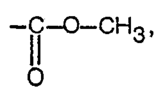, 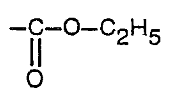, CN ou 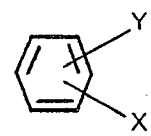,

X représentant un atome de chlore ou un groupe nitro, alkyle 1 à 4 C ou méthoxy et Y représentant un atome d'hydrogène ou de chlore le remplacement partiel correspondant à un taux d'estérification de 10% à 90% des groupes carboxyliques dans le cas où A est un atome d'hydrogène ou un groupe méthyle ou phényle, caractérisés en ce que ce sont des esters sélectifs dans lesquels sont estérifiés, partiellement ou totalement, uniquement les groupes carboxyliques des chaînons acide D-glucuronique, et les sels alcalins, alcalino-terreux, de magnésium, d'ammonium quaternaire et d'amine desdits esters.

2. Esters d'héparine dérivant de l'héparine par le remplacement partiel des groupements carboxyliques de l'héparine par des groupes de formule:

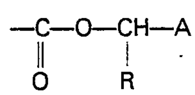
(I)

dans laquelle R est un atome d'hydrogène ou un groupe méthyle et A est un atome d'hydrogène ou un groupe méthyle, phényle,

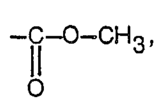, 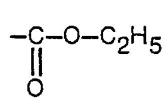, CN ou 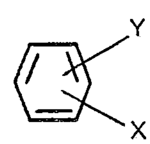,

X représentant un atome de chlore ou un groupe nitro, alkyle 1 à 4 C ou méthoxy et Y représentant un

atome d'hydrogène ou de chlore, le remplacement partiel correspondant à un taux d'estérisation de 10% à 90% des groupes carboxyliques dans le cas où A est un atome d'hydrogène ou un groupe méthyle ou phényle, caractérisés en ce que ce sont des esters sélectifs dans lesquels sont estérifiés, partiellement ou totalement, uniquement les groupes carboxyliques des chaînons acide D-glucuronique et acide L-iduronique non sulfaté, et les sels alcalins, alcalino-terreux, de magnésium, d'ammonium quaternaire et d'amine desdits esters.

3. Esters d'héparine dérivant de l'héparine par le remplacement partiel des groupements carboxyliques de l'héparine par des groupes de formule:

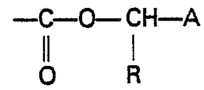 (I)

dans laquelle R est un atome d'hydrogène ou un groupe méthyle et A est un atome d'hydrogène ou un groupe méthyle, phényle,

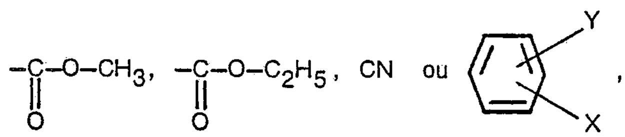
, CN ou ,

X représentant un atome de chlore ou un groupe nitro, alkyle 1 à 4 C ou méthoxy et Y représentant un atome d'hydrogène ou de chlore, le remplacement partiel correspondant à un taux d'estérification de 10% à 90% des groupes carboxyliques dans le cas où A est un atome d'hydrogène ou un groupe méthyle ou phényle, caractérisés en ce que ce sont des esters sélectifs dans lesquels sont estérifiés, partiellement ou totalement, uniquement les groupes carboxyliques des chaînons acide L-iduronique non sulfaté et acide L-iduronique sulfaté, et les sels alcalins, alcalino-terreux, de magnésium, d'ammonium quaternaire et d'amine desdits esters.

4. Esters d'héparine dérivant de l'héparine par le remplacement partiel des gorupements carboxyliques de l'héparine par des groupes de formule:

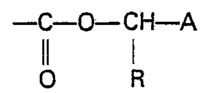 (I)

dans laquelle R est un atome d'hydrogène ou un groupe méthyle et A est un atome d'hydrogène ou un groupe méthyle, phényle,

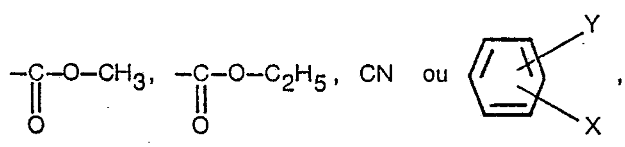
, CN ou ,

X representant un atome de chlore ou un groupe nitro, alkyle 1 à 4 C ou méthoxy et Y représentant un atome d'hydrogène ou de chlore, le remplacement partiel correspondant à un taux d'estérification de 10% à 90% des groupes carboxyliques dans le cas où A est un atome d'hydrogène ou un groupe méthyle ou phényle, caractérisés en ce que ce sont des esters sélectifs dans lesquels sont estérifiés, partiellement ou totalement, uniquement les groupes carboxyliques des chaînons acide L-iduronique sulfaté, et les sels alcalins alcalino-terreux, de magnésium, d'ammonium quaternaire et d'amine desdits esters.

5. Esters d'héparine selon l'une quelconque des revendications 1 à 4, caractérisés en ce que dans les groupes de formule (I), R est un atome d'hydrogène et A est un groupe

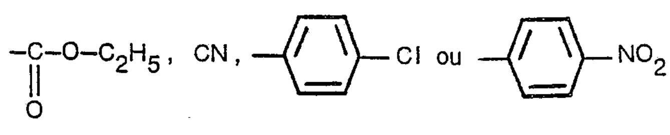
, CN , ou

et les sels alcalins, alcalino-terreux, de magnésium, d'ammonium quaternaire et d'amine desdits esters.

6. Sels alcalins d'esters selon l'une quelconque des revendications 1 à 5, caractérisés en ce que le cation alcalin est le sodium.

7. Sels alcalino-terreux d'esters selon l'une quelconque des revendications 1 à 5, caractérisés en ce que le cation alcalino-terreux est le calcium.

8. Sels de magnésium d'esters selon l'une quelconque des revendications 1 à 5.

9. Sels d'ammonium quaternaire d'esters selon l'une quelconque des revendications 1 à 5, caractérisés en ce que le cation ammonium quaternaire est le cation benzéthonium.

10. Procédé de préparation des composés selon la revendication 1, caractérisé en ce que:

1°) On prépare un sel acide d'ammonium quaternaire de l'héparine dans lequel sont salifiés, outre les groupes sulfate, uniquement les groupes carboxyliques des chaînons acide D-glucuronique, en faisant agir un sel d'ammonium quaternaire sur l'héparine, en milieu aqueux, à un pH compris entre 3 et 4,

2°) On fait réagir ledit sel acide d'ammonium quaternaire de l'héparine avec un dérivé halogéné de formule:

$$\begin{array}{c}\text{Hal—CH—A}\\ |\\ \text{R}\end{array} \qquad \text{(III)}$$

dans laquelle Hal est un atome de chlore, de brome ou d'iode et R et A ont les mêmes significations que dans la formule (I) de la revendication 1, en solution ou en suspension dans un solvant inerte, à une température comprise entre −20°C et +60°C.

11. Procédé de préparation des composés selon la revendication 2, caractérisé en ce que:

1°) On prépare un sel acide d'ammonium quaternaire de l'héparine dans lequel sont salifiés, outre les groupes sulfate, uniquement les groupes carboxyliques des chaînons acide D-glucuronique et acide L-iduronique non sulfaté, en faisant agir un sel d'ammonium quaternaire sur l'héparine, en milieu aqueux, à un pH compris entre 2 et 2,5, puis en neutralisant sélectivement les groupes carboxyliques des chaînons acide D-glucuronique et acide L-iduronique non sulfaté du produit ainsi obtenu par addition d'une quantité calculée d'hydroxyde d'ammonium quaternaire en milieu diméthylformamide,

2°) On fait réagir ledit sel acide d'ammonium quaternaire de l'héparine avec un dérivé halogéné de formule:

$$\begin{array}{c}\text{Hal—CH—A}\\ |\\ \text{R}\end{array} \qquad \text{(III)}$$

dans laquelle Hal est un atome de chlore, de brome ou d'iode et R et A ont les mêmes significations que dans la formule (I) de la revendication 2, en solution ou en suspension dans un solvant inerte, à une température comprise entre −20°C et +60°C.

12. Procédé de préparation des composés selon la revendication 3, caractérisé en ce qu'on fait réagir un alcool de formule:

$$\begin{array}{c}\text{HO—CH—A}\\ |\\ \text{R}\end{array} \qquad \text{(II)}$$

dans laquelle R et A ont les mêmes significations que dans la formule (I) de la revendication 3, avec l'héparine, en milieu aqueux, en présence d'un agent de condensation du type carbodi-imide soluble dans l'eau, le pH du milieu étant de 2 à 3.

13. Procédé de préparation des composés selon la revendication 4, caractérisé en ce que l'on fait réagir un alcool de formule:

$$\begin{array}{c}\text{HO—CH—A}\\ |\\ \text{R}\end{array} \qquad \text{(II)}$$

dans laquelle R et A ont les mêmes significations que dans la formule (I) de la revendication 4, avec l'héparine, en milieu aqueux, en présence d'un agent de condensation du type carbodiimide soluble dans l'eau, le pH du milieu étant de 3,5 à 4,5.

**Patentansprüche**

1. Heparinester, abgeleitet von Heparin durch teilweisen Ersatz der Carboxylgruppen des Heparins durch Gruppen der Formel

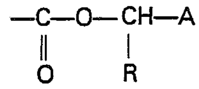 (I)

worin R ein Wasserstoffatom oder eine Methylgruppe ist und A ein Wasserstoffatom oder eine Methyl-, Phenyl-,

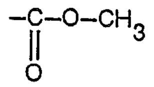, 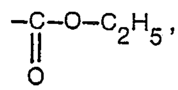, CN oder 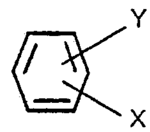,

gruppe ist, wobei X ein Chloratom oder eine Nitrogruppe, Alkylgruppe mit 1 bis 4 Kohlenstoffatomen oder Methoxy bedeutet und Y ein Wasserstoff- oder Chloratom bedeutet, wobei der teilweise Ersatz einem Veresterungswert von 10% bis 90% der Carboxylgruppen im Falle, wo A ein Wasserstoffatom oder eine Methyl- oder Phenylgruppe ist, entspricht, dadurch gekennzeichnet, daß es ausgewählte Ester sind, worin teilweise oder ganz nur die Carboxylgruppen der D-Glucuronsäure-Verettungen verestert sind und die Alkali-, Erdalkali-, Magnesium-, quaternären Ammonium- und Aminsalz dieser Ester.

2. Heparinester, abgeleitet von Heparin durch teilweisen Ersatz der Carboxylgruppen des Heparins durch Gruppen der Formel

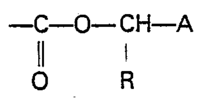 (I)

worin R ein Wasserstoffatom oder eine Methylgruppe ist und A ein Wasserstoffatom oder eine Methyl-, Phenylgruppe oder Gruppen

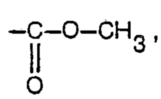, 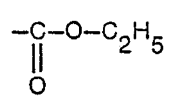, CN oder 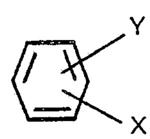,

ist, wobei X ein Chloratom oder eine Nitrogruppe, eine Alkylgruppe mit 1 bis 4 Kohlenstoffatomen oder Methoxy darstellt und Y ein Wasserstoff- oder Chloratom bedeutet, wobei der teilweise Ersatz einem Veresterungswert von 10 bis 90% der Carboxylgruppen im Fall, wo A ein Wasserstoffatom oder eine Methyl- oder Phenylgruppe ist, entspricht, dadurch gekennzeichnet, daß es ausgewählte Ester sind, worin teilweise oder ganz lediglich die Carboxylgruppen der D-Glucuronsäure- und nicht-sulfatierten L-Iduronsäure-Verkettungen verestert sind und die Alkali-, Erdalkali- Magnesium-, quaternären Ammonium- und Aminsalze dieser Ester.

3. Heparinester, abgeleitet von Heparin durch teilweisen Ersatz der Carboxylgruppen des Heparins durch Gruppen der Formel

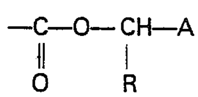 (I)

worin R ein Wasserstoffatom oder eine Methylgruppe ist und A ein Wasserstoffatom oder eine Methyl-, Phenylgruppe oder

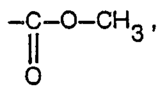, 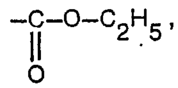, CN oder 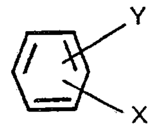,

ist, wobei X ein Chloratom oder eine Nitro- oder Alkylgruppe mit 1 bis 4 Kohlenstoffatomen oder Methoxy bedeutet und Y ein Wasserstoff- oder Chloratom darstellt, wobei der teilweise Ersatz einem Veresterungswert von 10 bis 90 der Carboxylgruppen im Falle, wo A ein Wasserstoffatom oder eine Methyl- oder Phenylgruppe ist, entspricht, dadurch gekennzeichnet, daß es ausgewählte Ester sind, worin teilweise oder ganz lediglich die Carboxylgruppen der nicht-sulfatierten L-Iduronsäure- und sulfatierten L-Iduronsäure-Verkettungen verestert sind und die Alkali-, Erdalkali-, Magnesium-, quaternären Ammonium- und Aminsalze dieser Ester.

4. Heparinester, abgeleitet von Heparin durch teilweisen Ersatz der Carboxylgruppen des Heparins durch Gruppen der Formel

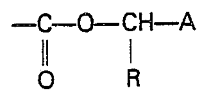 (I)

worin R ein Wasserstoffatom oder eine Methylgruppe ist und A ein Wasserstoffatom oder eine Methyl- oder Phenylgruppe oder

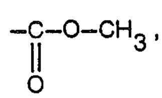, 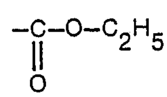, CN oder 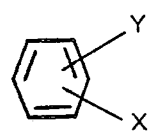,

ist, wobei X ein Chloratom oder eine Nitro- oder Alkylgruppe mit 1 bis 4 Kohlenstoffatomen oder Methoxy darstellt und Y ein Wasserstoff- oder Chloratom bedeutet, wobei der teilweise Ersatz einem Veresterungswert von 10 bis 90% der Carboxylgruppen im Falle, wo A ein Wasserstoffatom oder eine Methyl- oder Phenylgruppe bedeutet, entspricht, dadurch gekennzeichnet, daß es ausgewählte Ester sind, worin teilweise oder ganz lediglich die Carboxylgruppen der sulfatierten L-Iduronsäure-Verkettungen verestert sind, und die Alkali-, Erdalkali-, Magnesium-, quaternären Ammonium- und Aminsalze dieser Ester.

5. Heparinester gemäß einem der Ansprüche 1 bis 4, dadurch gekennzeichnet, daß in den Gruppen der Formel I R ein Wasserstoffatom ist und A eine Gruppe

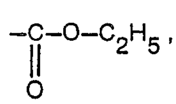, CN , 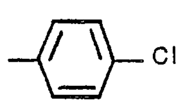 oder 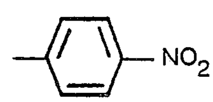

ist und die Alkali-, Erdalkali-, Magnesium-, quaternären Ammonium und Aminsalze dieser Ester.

6. Alkalisalze der Ester gemäß einem der Ansprüche 1 bis 5, dadurch gekennzeichnet, daß das Alkali-Kation Natrium ist.

7. Erdalkalisalze der Ester gemäß einem der Ansprüche 1 bis 5, dadurch gekennzeichnet, daß das Erdalkali-Kation Calcium ist.

8. Magnesiumsalze der Ester gemäß einem der Ansprüche 1 bis 5.

9. Quaternäre Ammoniumsalze der Ester gemäß einem der Ansprüche 1 bis 5, dadurch gekennzeichnet, daß das quaternäre Ammonium-Kation das Benzethonium-Kation ist.

10. Verfahren zur Herstellung der Verbindungen gemäß Anspruch 1, dadurch gekennzeichnet, daß man:

1.) ein saures quaternäres Ammoniumsalz des Heparins herstellt, worin außer den Sulfatgruppen lediglich die Carboxylgruppen der D-Glucuronsäure-Verkettungen in Salz überführt werden, indem ein quaternäres Ammoniumsalz auf das Heparin in wässrigem Milieu bei einem pH zwischen 3 und 4 einwirken gelassen wird,

2.) man setzt dieses saure quaternäre Ammoniumsalz des Heparins mit einem halogenierten Derivat der Formel

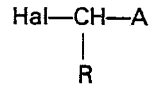 (III)

worin Hal ein Chloratom bromatom oder Jodatom ist und R und A dieselben Bedeutungen wie in Formel I von Anspruch 1 haben, in Lösung oder in Suspension in einem inerten Lösungsmittel bei einer Temperatur zwischen −20°C und +60°C um.

11. Verfahren zur Herstellung der Verbindungen gemäß Anspruch 2, dadurch gekennzeichnet, daß man:

1.) ein saures quaternäres Ammoniumsalz des Heparins herstellt, worin außer den Sulfatgruppen lediglich die Carboxylgruppen der D-Glucuronsäure- und nicht-sulfatierten L-Iduronsäure-Verkettungen verestert sind, indem man ein quaternäres Ammoniumsalz auf das Heparin in wässrigem Milieu bei einem pH zwischen 2 und 2,5 einwirken läßt und indem man dann selektiv die Carboxylgruppen der D-Glucuronsäure- und nicht-sulfatierten L-Iduronsäure-Verkettungen des so erhaltenen Produktes durch Zugabe einer berechneten Menge von quaternärem Ammoniumhydroxid in dimethylformamid-Milieu neutralisiert,

2.) daß man dieses saure quaternäre Ammoniumsalz des Heparins mit einem halogenierten Derivat der Formel

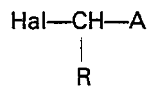 (III)

worin Hal ein Chlor-, Brom- oder jodatom ist und R und A dieselben Bedeutungen wie in Formel I von Anspruch 2 haben, in Lösung oder in Suspension in einem inerten Lösungsmittel bei einer Temperatur zwischen −20°C und +60°C reagieren läßt.

12. Verfahren zur Herstellung der Verbindungen gemäß Anspruch 3, dadurch gekennzeichnet, daß man einen Alkohol der Formel

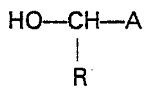 (II)

worin R und A dieselben Bedeutungen wie in Formel I von Anspruch 3 haben, mit Heparin in wässrigem Milieu in Gegenwart eines Kondensationsmittels vom Typ Carbodiimid, Löslich in Wasser, zur Reaktion bringt, wobei das pH des Milieus von 2 bis 3 beträgt.

13. Verfahren zur Herstellung der Verbindungen gemäß Anspruch 4, dadurch gekennzeichnet, daß man einen Alkohol der Formel

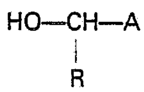 (II)

worin R und A dieselben Bedeutungen wie in Formel I von Anspruch 4 haben, mit Heparin in wässrigem Milieu in Gegenwart eines Kondensationsmittels vom Typ Carbodiimid, löslich in Wasser, zur Reaktion bringt, wobei das pH des Milieus von 3,5 bis 4,5 beträgt.

**Claims**

1. Heparin esters derived from heparin by partial replacement of the carboxyl groups of heparin by groups of the formula:

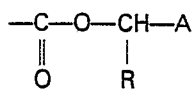 (I)

in which R is a hydrogen atom or a methyl group and A is a hydrogen atom or a methyl, phenyl,

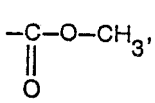, 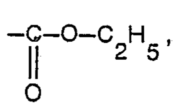, CN or 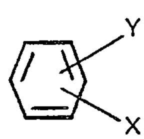

group, where X represents a chlorine atom or a nitro, alkyl (1 to 4 C) or methoxy group and Y represents a hydrogen atom or chlorine atom, the partial replacement corresponding to a degree of esterification of the carboxyl groups of 10% to 90% in the case where A is a hydrogen atom or a methyl or phenyl group, characterised in that these are selective esters in which are partially or completely esterified only the carboxyl groups of the D-glucuronic acid chain members, and the alkali metal, alkaline earth metal, magnesium, quaternary ammonium and amine salts of the said esters.

2. Heparin esters derived from heparin by partial replacement of the carboxyl groups of heparin by groups of the formula:

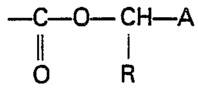 (I)

in which R is a hydrogen atom or a methyl group and A is a hydrogen atom or a methyl, phenyl,

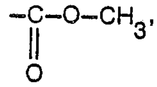, 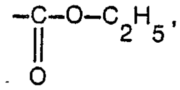, CN or 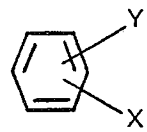

group, where X represents a chlorine atom or a nitro, alkyl (1 to 4 C) or methoxy group and Y represents a hydrogen atom or chlorine atom, the partial replacement corresponding to a degree of esterification of the carboxyl groups of 10% to 90% in the case where A is a hydrogen atom or a methyl or phenyl group, characterised in that these are selective esters in which are partially or completely esterified only the carboxyl groups of the D-glucuronic acid chain members, and of the non-sulphated L-iduronic acid chain members, and the alkali metal, alkaline earth metal, magnesium, quaternary ammonium and amine salts of the said esters.

3. Heparin esters derived from heparin by partial replacement of the carboxyl groups of heparin by groups of the formula:

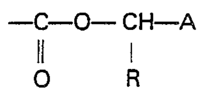 (I)

in which R is a hydrogen atom or a methyl group and A is a hydrogen atom or a methyl, phenyl,

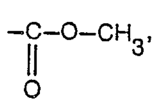, 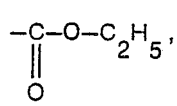, CN or 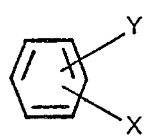

group, where X represents a chlorine atom or a nitro, alkyl (1 to 4 C) or methoxy group and Y represents a hydrogen atom or chlorine atom, the partial replacement corresponding to a degree of esterification of the carboxyl groups of 10% to 90% in the case where A is a hydrogen atom or a methyl or phenyl group, characterised in that these are selective esters in which are partially or completely esterified only the carboxyl groups of the non-sulphated L-iduronic acid chain members and sulphated L-iduronic acid chain members, and the alkali metal, alkaline earth metal, magnesium, quaternary ammonium and amine salts of the said esters.

4. Heparin esters derived from heparin by partial replacement of the carboxyl groups of heparin by groups of the formula:

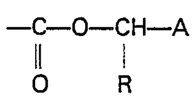 (I)

in which R is a hydrogen atom or a methyl group and A is a hydrogen atom or a methyl, phenyl,

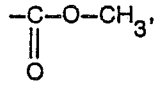, 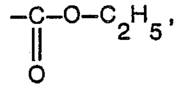, CN or 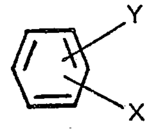

group, where X represents a chlorine atom or a nitro, alkyl (1 to 4 C) or methoxy group and Y represents a hydrogen atom or chlorine atom, the partial replacement corresponding to a degree of esterification of the carboxyl groups of 10% to 90% in the case where A is a hydrogen atom or a methyl or phenyl group, characterised in that these are selective esters in which are partially or completely esterified only the carboxyl groups of the sulphated L-iduronic acid chain members, and the alkali metal, alkaline earth metal, magnesium, quaternary ammonium and amine salts of the said esters.

5. Heparin esters according to any one of Claims 1 to 4, characterised in that in the groups of formula (I), R is a hydrogen atom and A is a

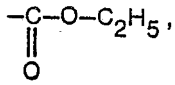, CN, 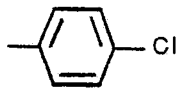 or 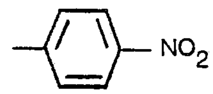

group, and the alkali metal, alkaline earth metal, magnesium, quaternary ammonium and amine salts of the said esters.

6. Alkali metal salts of esters according to any one of Claims 1 to 5, characterised in that the alkali metal cation is sodium.

7. Alkaline earth metal salts of esters according to any one of Claims 1 to 5, characterised in that the alkaline earth metal cation is calcium.

8. Magnesium salts of esters according to any one of Claims 1 to 5.

9. Quaternary ammonium salts of esters according to any one of Claims 1 to 5, characterised in that the quaternary ammonium cation is the benzethonium cation.

10. Process for the preparation of the compounds according to Claim 1, characterised in that:

1) an acid quaternary ammonium salt of heparin is prepared, in which, other than the sulphate groups, only the carboxyl groups of the D-glucuronic acid chain members are salified, by reacting a quaternary ammonium salt with heparin in an aqueous medium at a pH of between 3 and 4, and

2) the said acid quaternary ammonium salt of heparin is reacted with a halogen derivative of the formula:

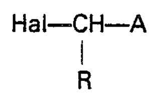 (III)

in which Hal is a chlorine, bromine or iodine atom and R and A have the same meanings as in formula (I) of Claim 1, in solution or suspension in an inert solvent at a temperature of between −20°C and +60°C.

11. Process for the preparation of the compounds according to Claim 2, characterised in that:

1) an acid quaternary ammonium salt of heparin, in which, other than the sulphate groups, only the carboxyl groups of the D-glucuronic acid chain members and non-sulphated L-iduronic acid chain members are salified, is prepared by reacting a quaternary ammonium salt with heparin in an aqueous medium at a pH of between 2 and 2.5 and then selectively neutralising the carboxyl groups of the D-glucuronic acid chain members and non-sulphated L-iduronic acid chain members of the product thus obtained by adding a calculated quantity of quaternary ammonium hydroxide in a dimethylformamide medium, and

2) the said acid quaternary ammonium salt of heparin is reacted with a halogen derivative of the formula:

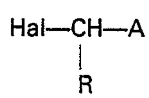 (III)

in which Hal is a chlorine, bromine or iodine atom and R and A have the same meanings as in formula (I) of Claim 2, in solution or suspension in an inert solvent at a temperature of between −20°C and +60°C.

12. Process for the preparation of the compounds according to Claim 3, characterised in that an alcohol of the formula:

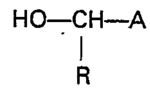

(II)

in which R and A have the same meanings as in formula (I) of Claim 3, is reacted with heparin in an aqueous medium in the presence of a water-soluble condensation agent of the carbodiimide type, the pH of the medium being from 2 to 3.

13. Process for the preparation of the compounds according to Claim 4, characterised in that an alcohol of the formula:

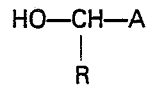

(II)

in which R and A have the same meanings as in formula (I) of Claim 4, is reacted with heparin in an aqueous medium in the presence of a water-soluble condensation agent of the carbodiimide type, the pH of the medium being from 3.5 to 4.5.